# EUROPEAN PATENT SPECIFICATION

(11) **EP 0 315 092 B1**
(45) Date of publication and mention of the grant of the patent: **01.12.1993**
(21) Application number: 88118060.8
(22) Date of filing: 28.10.1988
(51) Int. Cl.: G01N 33/38, G01N 7/14, G01N 15/06

(54) **A method of determining the quality of an air bubble system in fresh concrete**
Verfahren zum Qualitätsnachweis eines Luftbläschensystems im frischen Beton
Méthode pour déterminer la qualité d'un système de bulles d'air dans le béton frais

(30) Priority: 03.11.1987 DK 5749/87
(43) Date of publication of application: 10.05.1989
(73) Proprietor: DANSK BETON TEKNIK A/S, DK-2900 Hellerup (DK)
(72) Inventor: Jensen, Bent Jorgen, DK-2900 Hellerup (DK)
(74) Representative: Koepsell, Helmut, Dipl.-Ing.

(56) References cited:
- GB-A- 714 556
- US-A- 4 043 178
- US-A- 4 270 381
- SOVIET INVENTIONS ILLUSTRATED, Week C27, 13 August 1980, no. F8787, Derwent, London (GB)#
- SOVIET INVENTIONS ILLUSTRATED, Week E36, 20 October 1982, no. L9468, Derwent, London (GB)#

## Description

This invention relates to a method of determining the quality of an air bubble system in fresh concrete.

It is known that an air bubble system amounting to 4-8% in concrete can bring about an enhanced frost resistance in the cast concrete if the said system presents a fine pore structure, expressed, for instance, in terms of the air bubble system having a certain minimum value of specific surface or in terms of the presence of a certain minimum quantity of air bubbles having a maximum diameter, for instance 2,5% air presenting air bubbles of a diameter of 0,5 mm or less.

In SU-A-697.929 (Gosstroi Concrete) a method of determining the mean radius of air bubbles in concrete is disclosed. The difference in pressure before and after release of the entrained air bubbles is measured, and then a thermodynamic formula is used for the calculation of the mean radius, assuming the air bubbles all being equally sized.

Su-A-881.615 (Pylaev A Ya) discloses a method for the continuous registration of the total air volume in, for instance, samples of concrete.

The invention is based on the acknowledgement of the fact that the composition of the air bubble system in fresh concrete before casting is substantially identical to the composition of the system in the cast and hardened concrete.

The invention suggests a method of determining the quality of an air bubble system in fresh concrete, expressed, for instance, by means of the specific surface of the air bubble system, and the method is characterized by the fact that the air bubbles are forced out of the sample of the fresh concrete and are subjected to an analysis in which the expelled air quantity is registered as a function of time whereupon the quality of the air bubble system is expressed by means of the analysis result.

By means of this method one can predict the quality of a planned casting before it is carried out so that a sub-standard batch can be rejected while according to the known art one must first cast the construction concerned which upon hardening can be sampled and analysed and only then one can determine if the construction has to be rejected.

Experiments have shown that an especially suited embodiment of the method concerned consists in that the air bubbles in a container are expelled from the sample in an analysis medium by means of agitation and that the bubbles are transferred to a superposed screening medium in which they ascend being collected at the top of said medium and subjected to a registration as a function of time. The bigger bubbles will leave the analysis medium and ascend more quickly towards the surface of the screening medium than will the smaller air bubbles so that the time of passage through a given level in the screening medium of the individual bubbles will reflect the diameter of the air bubble concerned. A weighing may be a determination of the increase of the buoyancy of an immersed collection bell jar.

Expelling of the air bubbles of the sample may also be effected by centrifuging, vibration or other handling.

Experiments have revealed an advantage in using glycerol as an analysis medium and water as a screening medium. Other media like glycols, alcohols, salt solutions and sugar solutions may also be used.

By making use of the method concerned it has been shown that a prolonged time of mixing the fresh concrete gives rise to a high level of specific surface. The same is true of pumping and vibration of the concrete. The method concerned may therefore contribute to quality supervision and concrete production control.

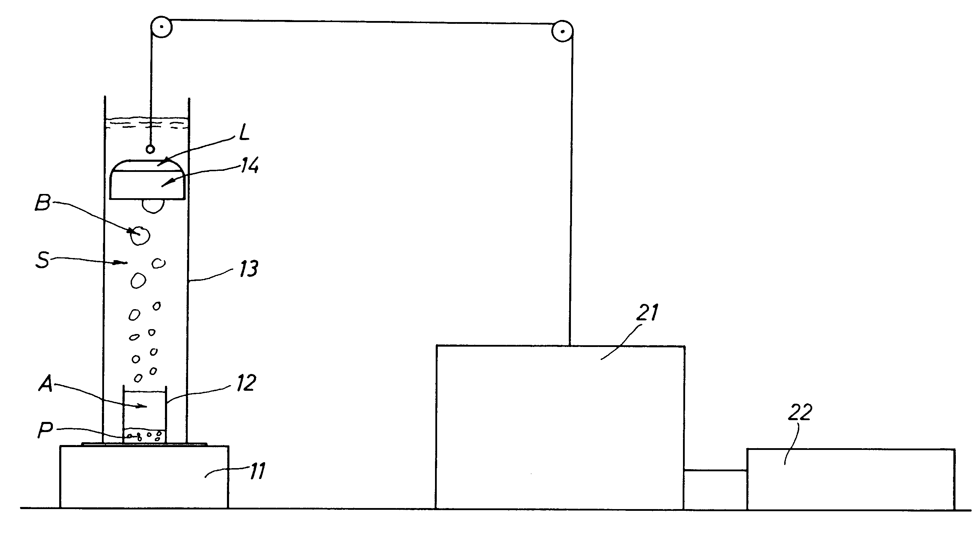

The invention is further illustrated in the following description, reference being had to the drawing of an apparatus for carrying out the method of the invention.

The apparatus shown comprises a magnetic agitator 11 on which is placed an analysis container 12 with a concrete sample P in an analysis medium A, and surrounded by a screening container 13 with a screening medium S. On top of the screening container 13 is placed a receptacle 14 for the collected air quantity L formed by the air bubbles B set free from the sample. The air quantity L is registered as a function of time by means of a digital scale 21 connected to a computer 22 having a printer.

### Method

A 10 ml sample is removed from the fresh concrete to be tested. The sample is placed in the analysis container 12, height 45 mm, diametre 50 mm, corresponding to a volume of approximately 100 ml.

To the sample is added an analysis medium, for instance glycerol the nature of which is to the effect that the air bubbles relieved by the agitation do not change their identity, for instance due to division, combining or other modifications of shape. The sample and the analysis medium are placed in the screening container 13 which is carefully Filled with water, and the whole setup is placed on the magnetic agitator 11.

By weighing the air quantity corresponding to the bubbles relieved as a function of time one calculates the specific surface of the air bubble system or another value, for instance the amount of air bubbles in the sample having a smaller diameter than a prescribed maximum diameter.

### Example 1

A cement mortar is prepared for use as a plastering coating on a concrete slab used as a lorry wash stand.

The latter is out-of-doors and should be safeguarded in the best possible way against frost injury. A water/cement ratio below 0,40 has therefore been specified and an air bubble generating agent should be used which produces an air content of the order of magnitude of 15-25% of the paste portion of the fresh cement mortar (the paste portion meaning to say water + cement + air), or 8-12% rckoned on the mortar quantity. The air content is measured as indicated in DS 423.11 (DS = Danish Standard Specification).

It is furthermore specified that the air bubble system in the finally hardened concrete should have a specific surface of at least 25 mm⁻¹ and a distance factor below 0,20 mm, both values being determined according to ASTM C 457.

A mortar having the following composition is furnished (per 1000 litres):

| | |
|---|---|
| Cement | 620 kg (197 litres) |
| Sand 0/4 mm | 1240 kg (473 litres) |
| Water | 240 kg (240 litres) |
| Air bubble generating agent | 1 kg, expected air production 90 litres |

According to the recipe this means to say the following values of the mortar prepared:

| | |
|---|---|
| Water/cement ratio: | 0,39 i.e. less than 0,40 |
| Air content: | 21% of paste volume |

The specified requirements are thus fulfilled according to the recipe.

### Known Art

A laboratory test casting comprinsing determination of the air contents of the fresh mortar and pore structure analysis according to ASTM C 457 of the hardened cement mortar gives the following results:
Air content determined according to DS 423.11:18 %
Structure analysis of hardened concrete according to ASTM C 457:

| | |
|---|---|
| Air content: | 16% |
| Specific surface: | 48 mm⁻¹ |
| Distance factor: | 0,13 mm |

The quality requirements are seen to be fulfilled.

### Determination According to the Invention

A determination of the specific surface of the air bubble system according to the method of the invention gives a value of 50 mm⁻¹. A later control determination of the specific surface of the air bubble system in a hardened mortar sample confirms this result.

### Example 2

Each batch of concrete for the construction of a bridge is checked by the method of the invention in a period of 2 casting days before and after the casting. The analysis results below are obtained leading to the rejection of batch No. 7. In connection with this rejection a laboratory control casting of a sample of the rejected concrete is carried out.

| **Batch No.** | **Fresh Concrete before Casting** | **Specific Surface mm⁻¹ in construction** | **Hardened Concrete Specific Surface mm⁻¹** |
|---|---|---|---|
| 1 | 34 | 34 | |
| 2 | 29 | 31 | 30 |
| 3 | 39 | 38 | |
| 4 | 37 | 40 | |
| 5 | 40 | 42 | 44 |
| 6 | 41 | 39 | |
| 7 | 20 | 22 | 22 |
| 8 | 35 | 39 | 34 |
| 9 | 28 | 33 | |
| 10 | 32 | 31 | |
| 11 | 30 | 30 | |
| 12 | 30 | 33 | 36 |
| 13 | 31 | 33 | |
| 14 | 34 | 34 | |
| 15 | 36 | 40 | 40 |
| 16 | 30 | 32 | |
| 17 | 42 | 41 | |
| 18 | 37 | 40 | |
| 19 | 30 | 35 | |
| 20 | 39 | 40 | |
| 21 | 28 | 28 | |
| 22 | 31 | 34 | |
| 23 | 39 | 42 | |
| 24 | 40 | 39 | |
| 25 | 42 | 46 | |
| 26 | 44 | 48 | 47 |
| 27 | 40 | 41 | |
| 28 | 38 | 40 | |
| 29 | 35 | 38 | |
| 30 | 41 | 40 | |

The above table directly demonstrates the usefulness of the method concerned for supervision of the quality of concrete.

The measuring of the air bubbles contained in the sample of the fresh concrete usually takes place within 30 min. Within this period of time practically all the air bubbles have been released from the sample and have been collected at the top of the measuring set up. At appropriate intervals during the period, for instance every 15 seconds, the amount of released air is registrated in an appropriate way, these registrations being used in the calculation of the quality of the sample - as shown in the following example 3.

### Example 3

Two samples, sample F 21 and sample E 3 of fresh concrete are examined according to the method of the present invention in order to establish whether or not the air bubble systems are of a sufficiently porous quality for protecting the concrete against damages, caused by frost. The hardened concrete, corresponding to the samples F 21 and E 3, are further examined according to ASTM C 457.

The following results are obtained:

### Sample F 21:

| **Analysis time** | **Buoyancy (g x 10⁻²)** |
|---|---|
| 0 sec. | 0 |
| 15 sec. | 4 |
| 30 sec. | 77 |
| 45 sec. | 92 |
| 60 sec | 97 |
| 90 sec. | 106 |
| 120 sec. | 111 |
| 3 min. | 116 |
| 4 min. | 118 |
| 5 min. | 120 |
| 7 min. | 122 |
| 10 min. | 124 |
| 15 min. | 127 |
| 20 min. | 129 |
| 25 min. | 130 |

The air content and the specific surface of the air bubbles in the sample F 21 (fresh concrete) are calculated using these results:

| | |
|---|---|
| Air content (air bubble diameter < 2 mm): | 2,8% by volume of concrete |
| Specific surface (air bubble diameter < 2 mm): | 18,0 mm⁻¹ |

According to ASTM C 457 the following results are obtained by analysing the hardened concrete, corresponding to the sample F 21:

| | |
|---|---|
| Air content (air bubble diameter < 2 mm): | 2,8% of concrete |
| Specific surface (air bubble diameter < 2 mm): | 17,9 mm⁻¹ |

The correspondance is seen to be very good.

### Sample E3:

| **Analysis time** | **Buoyancy (g x 10⁻²)** |
|---|---|
| 0 sec. | 0 |
| 15 sec. | 25 |
| 30 sec. | 34 |
| 45 sec. | 41 |
| 60 sec | 47 |
| 90 sec. | 58 |
| 120 sec. | 70 |
| 3 min. | 91 |
| 4 min. | 110 |
| 5 min. | 127 |
| 7 min. | 154 |
| 10 min. | 199 |
| 15 min. | 225 |
| 20 min. | 242 |
| 25 min. | 255 |
| 30 min. | 256 |

The following calculations are made:

| | |
|---|---|
| Air content (diameter < 2 mm): | 5,6% by volume of the concrete |
| Specific surface (diameter < 2 mm): | 35,8 mm⁻¹ |

The hardened concrete is subjected to analysis according to ASTM C 457:

| | |
|---|---|
| Air content (diameter < 2 mm): | 5,5% by volume of the concrete |
| Specific surface (diameter < 2 mm): | 35,8 mm⁻¹ |

Again the results obtained by the two methods are very much alike.

The air bubble system of the sample E 3 is of a good enough quality to protect the concrete against frost damages, whereas the sample F 21 must be rejected.

By using the method according to the invention the whole batch of fresh concrete corresponding to the sample F 21 can be rejected before casting it.

## Claims

1. A method of determining the quality of an air bubble system in fresh concrete, **characterized** by the fact that the air bubbles in a container are expelled from the sample of fresh concrete in an analysis medium by means of agitation and that the bubbles are transferred to a superposed screening medium in which they ascend being collected at the top of said medium and are subjected to an analysis in which the expelled air quantity is registered as a function of time whereupon the quality of the air bubble system is expressed by means of the analysis result, acknowledging the fact that the time of passage through a given level in the screening medium of the individual bubbles reflects the diameter of the air bubbles concerned.

2. A method of claim 1, **characterized** by the fact that the analysis medium is glycerol and that the screening medium is water.

3. A method according to claims 1-2, **characterized** by the fact that the registration of the expelled air quantity as a function of time is effected by weighing or by counting and measuring.

4. A method according to claim 3, **characterized** by the fact that the counting and measuring of the air bubbles take place visually, optically or opto-electrically.

## Patentansprüche

1. Verfahren zum Qualitätsnachweis eines Luftbläschensystems in frischem Beton, dadurch gekennzeichnet, daß die Luftbläschen in einem Behälter aus der Probe frischen Betons in einem Analysemedium mittels Schütteln ausgeschieden und die Luftbläschen an ein überlagertes Klassiermedium abgegeben werden, in welchem sie aufsteigen, und die Luftbläschen an der Oberfläche dieses Mediums gesammelt und einer Analyse unterworfen werden, bei welcher die ausgeschiedene Luftmenge als eine Funktion der Zeit festgehalten wird, wonach die Qualität des Luftbläschensystems mittels des Analyseergebnisses ausgedrückt wird, wobei der Tatsache Rechnung getragen wird, daß die Durchlaufzeit durch eine vorgegebene Standhöhe im Klassiermedium den Durchmesser der betroffenen Luftbläschen widerspiegelt.

2. Verfahren nach Anspruch 1, dadurch gekennzeichnet, daß das Analysemedium Glycerin und das Klassiermedium Wasser ist.

3. Verfahren nach den Ansprüchen 1 und 2, dadurch gekennzeichnet, daß das Festhalten der ausgeschiedenen Luftmenge als eine Funktion der Zeit durch Wiegen oder Zählen und Messen durchgeführt wird.

4. Verfahren nach Anspruch 3, dadurch gekennzeichnet, daß das Zählen und Messen der Luftbläschen visuell, optisch oder opto-elektrisch durchgeführt wird.

## Revendications

1. Une méthode pour déterminer la qualité d'un système à bulles d'air dans du béton frais, caractérisée en ce que les bulles d'air sont expulsées dans un récipient à partir de l'échantillon de béton frais dans un milieu d'analyse par agitation et en ce que les bulles sont transférées dans un milieu de protection placé au dessus, dans lequel elles s'élèvent en étant recueillies aus sommet de ce milieu, et sont soumises à une analyse dans laquelle la quantité d'air expulsé est enregistrée en tant que fonction du temps, à partir de laquelle la qualité du système de bulles d'air est exprimée au moyen du résultat de l'analyse, constatation faite que la durée du passage à travers un niveau déterminé dans le milieu de protection des bulles individuelles représente le diamètre des bulles d'air concernées.

2. Une méthode selon la revendication 1, caractérisée en ce que le milieu d'analyse est de la glycérine et que le milieu de protection est de l'eau.

3. Une méthode selon les revendications 1 et 2, caractérisée en ce que l'enregistrement de la quantité d'air expulsé en tant que fonction du temps est effectué par pesée ou par comptage et mesure.

4. Une méthode selon la revendication 3, caractérisé en ce que le comptage et la mesure des bulles d'air est réalisé par voie visuelle, optique ou opto-électronique.
